# EUROPÄISCHE PATENTSCHRIFT

(11) **EP 3 280 372 B1**
(45) Veröffentlichungstag und Bekanntmachung des Hinweises auf die Patenterteilung: **28.11.2018**
(21) Anmeldenummer: 17733451.3
(22) Anmeldetag: 27.06.2017
(51) Int. Cl.: A61F 13/08, A61F 15/00

(54) **AN- UND AUSZIEHHILFE FÜR KOMPRESSIONSSTRÜMPFE**
AID FOR PULLING ON AND TAKING OFF COMPRESSION STOCKINGS
DISPOSITIF D'ASSISTANCE POUR ENFILER ET ÔTER DES BAS DE CONTENTION

(30) Priorität: 28.06.2016 DE 102016111834
(43) Veröffentlichungstag der Anmeldung: 14.02.2018
(73) Patentinhaber: Junge, Dirk, 86981 Kinsau (DE)
(72) Erfinder: Junge, Dirk, 86981 Kinsau (DE)
(74) Vertreter: Lohr, Jöstingmeier & Partner
(86) Internationale Anmeldenummer: PCT/EP2017/065855
(87) Internationale Veröffentlichungsnummer: WO 2018/002050

(56) Entgegenhaltungen:
- WO-A1-99/44558
- DE-A1-102004 032 555
- DE-A1-102004 047 248
- DE-A1-102012 100 122
- DE-U1- 9 401 036

## Beschreibung

### Technisches Gebiet

Die Erfindung betrifft eine An- und Ausziehhilfe für Kompressionsstrümpfe mit wenigstens einem ersten folienartigen Streifen, wobei auf den ersten Streifen ein zweiter Streifen derart aufgesetzt ist, dass der zweite Streifen mit dem ersten Streifen eine Tasche mit einem in Längsrichtung weisenden geschlossenen Ende bildet. Die Tasche hat an dem dem geschlossenen Ende gegenüberliegenden Ende eine Öffnung in Längsrichtung und die Öffnung wird vom ersten Streifen und vom zweiten Streifen in Längsrichtung überragt und das geschlossene Ende der Tasche unterteilt den ersten Streifen in zwei Abschnitte.

### Stand der Technik

Kompressionsstrümpfe, dienen dazu die Venen und das Lymphsystem im Bereich des Beines zu entlasten und werden zur Behandlung und Vermeidung von Thrombosen eingesetzt, entsprechend werden sie auch als Anti-Thrombosestrümpfe oder Stützstrümpfe bezeichnet. Kompressionsstrümpfe bestehen in der Regel aus einem elastischen Material, das beim Anziehen gedehnt wird, damit ein angezogener Strumpf einen radial nach innen weisender Druck auf das Bein ausübt. Dieses Dehnen macht das Anziehen von Kompressionsstrümpfen insbesondere für ältere Menschen mit eingeschränkter Beweglichkeit und Kraft schwierig. Um das Anziehen von Kompressionsstrümpfen zu erleichtern, sind verschiedene Anziehhilfen vorgeschlagen worden:
Aus der EP 0 332 837 B1 ist eine Anziehhilfe mit wenigstens vier in etwa parallelen Streben bekannt. An einem ersten Ende sind die Streben durch einen ersten Bügel und an dem anderen Ende durch einen weiteren Bügel miteinander verbunden. Der Kompressionsstrumpf wird auf die Streben aufgezogen, dabei gedehnt, und kann dann angezogen werden. Anschließend werden die Streben aus dem Strumpf gezogen.

Aus der DE 10 2004 047 248 A1 ist eine An- und Ausziehhilfe für Kompressionsstrümpfe bekannt. Die An- und Ausziehhilfe besteht aus einer gut gleitenden Folie, die an einem Ende zu einem sackartigen Ende geformt ist. An dem anderen Ende hat die Folie die Form einer langstreckten Zunge. Zum Anziehen wird die Zunge einmal umgefaltet, so dass zwei Lagen der Zunge aufeinanderliegen. Die beiden Lagen der Folie werden unter Fußsohle und um die Ferse herum nach hinten geführt. Dazu muss die Folie mit den Zehen fixiert werden. Nun wird ein Kompressionsstrumpf über das Bein und die von den Zehen gehaltene Folie bis etwa unterhalb des Knies gezogen, wobei er durch die geringe Haftreibung der Folie vergleichsweise einfach über den Fuß und die Ferse zu ziehen ist. Anschließend können die Zehen die Folie freigeben und sie wird an einem ihrer aus dem Kompressionsstrumpf hängenden freien Enden aus dem Kompressionsstrumpf herausgezogen.

Die DE 100 11 727 A1 offenbart eine Anziehhilfe für Strümpfe aus zwei übereinander angeordneten Lagen, die dadurch eine Tasche bilden, wobei die Enden der Lagen am geschlossen Ende der Tasche enden. Zwischen den beiden Lagen ist ein Zugband angeordnet, welches am geschlossenen Ende der Tasche mit den Enden der beiden Lagen verbunden ist, um die Anziehhilfe nach dem Überstreifen eines Strumpfes über einen Fuß und den daran angrenzenden Unterschenkel nach hinten aus dem Strumpf herausziehen zu können.

In der DE 10 2012 100 122 ist die Anziehhilfe der eingangs beschriebenen Art beschrieben. Diese ermöglicht ein besonders einfaches Anziehen der Stützstrümpfe, jedoch muss zum Ausziehen eine separate Ausziehhilfe verwendet werden.

### Darstellung der Erfindung

Der Erfindung liegt die Aufgabe zugrunde, die eingangs beschriebene Anziehhilfe so weiterzubilden, dass sie auch als Ausziehhilfe verwendet werden kann.

Diese Aufgabe wird durch eine An- und Ausziehhilfe nach Anspruch 1 gelöst. Vorteilhafte Ausgestaltungen der Erfindung sind in den Unteransprüchen angegeben.

Die An- und Ausziehhilfe für Kompressionsstrümpfe hat wenigstens einen ersten folienartigen Streifen, z.B. aus einem textilen Material, wie es zur Herstellung von Spinnakern im Segelsport oder zu Herstellung von Gleitschirmen im Fallschirmsport üblicherweise verwendet wird. Diese Materialen haben eine sehr hohe Zugfestigkeit in Längsrichtung und eine geringe Haft- und Gleitreibung auf sich selbst und den üblichen für Kompressionsstrümpfe verwendeten elastischen Textilien. Auf den ersten Streifen ist ein zweiter Streifen derart aufgesetzt, dass er mit dem ersten Streifen eine Tasche mit einem in Längsrichtung weisenden geschlossenen Ende bildet. Der zweite Streifen ist vorzugsweise aus dem gleichen Material wie der erste Streifen. Die Verbindung zwischen den beiden Streifen kann durch nähen und/oder kleben erfolgen. Natürlich ist bei thermoplastischen Materialen auch ein Verschweißen sinnvoll.

Die Tasche hat eine Öffnung in Längsrichtung und entsprechend ein geschlossenes Ende und das geschlossene Ende der Tasche unterteilt den ersten Streifen in zwei Abschnitte, wobei die Tasche auf dem zweiten Abschnitt angeordnet ist.

Vorzugsweise wird die Öffnung vom ersten Streifen und vom zweiten Streifen in Längsrichtung überragt. Wie nachfolgend näher beschrieben, wird dadurch das spätere Entfernen der An- und Ausziehhilfe nach dem Anziehen eines Stützstrumpfs erleichtert.

Vorzugsweise ist der der erste Abschnitt länger ist als der zweite Abschnitt um das Herausziehen der An- und Ausziehhilfe zu erleichtern.

Der erste Abschnitt hat bevorzugt wenigsten einen ersten Unterabschnitt, an dem seine beiden Längsseiten miteinander verbunden sind, so dass der Unterabschnitt einen Hohlkörper ausbildet. Der Hohlkörper ist bevorzugt tunnelartig, d.h. er bildet vorzugsweise eine beidseits offene Röhre, die sich in Richtung der Streifenlängsachse erstreckt. Der Hohlkörper kann sich beispielsweise in Richtung des freien Ende des ersten Abschnitts verjüngen, d.h. der freie Durchmesser des (angenommen) aufgespannten Hohlkörpers nimmt dann in dieser Richtung ab.

Diese An- und Ausziehhilfe ermöglicht es einen Stützstrumpf sehr einfach anzuziehen, indem man den ersten Abschnitt im Bereich des geschlossenen Endes der Tasche unter den zweiten Abschnitt umschlägt und das freie Ende des zweiten Streifens in die Tasche umschlägt. Nun kann ein Fuß mit den Zehen voran in die Tasche eingeschoben werden. Anschließend wird der Stützstrumpf über den Fuß und das Bein geschoben, wobei die An- und Ausziehhilfe zunächst zwischen dem Bein und dem Stützstrumpf verbleibt und das Hochschieben des Stützstrumpfs erleichtert, weil der Stützstrumpf auf dem Material der An- und Ausziehhilfe gut gleitet. Nachdem der Stützstrumpf seine Sollposition erreicht hat, d.h. den vorgesehenen Bereich des Beines faltenfrei umschließt, wird die An-und Ausziehhilfe wieder entfernt, dazu genügt es, an dem freien Ende des ersten Abschnitts zu ziehen. Dann gleitet der zweite Streifen über sich selbst, so dass die Tasche um die Zehen herum gleitet. Anschließend wird einfach so lange an dem freien Ende des ersten Abschnitts gezogen, bis die An- und Ausziehhilfe vollständig aus dem Spalt zwischen Bein und Stützstrumpf entfernt wurde. Dieses Verfahren funktioniert sowohl bei Stützstrümpfen mit geschlossener als auch bei solchen mit offener Fußspitze.

Noch einfacher funktioniert das Anziehen von Stützstrümpfen mit offener Fußspitze unter Verwendung der An- und Ausziehhilfe. Dazu wird wie bei dem zuvor beschriebenen Verfahren zunächst der erste Abschnitt im Bereich des geschlossenen Endes der Tasche unter den zweiten Abschnitt umgeschlagen und das freie Ende des zweiten Streifens in die Tasche umgeschlagen, d.h. eingelegt. Nun kann der Fuß mit den Zehen voran in die Tasche eingeschoben werden. Anschließend wird der Fuß mit der Tasche in den Hohlkörper eingeschoben. Der anzuziehende Stützstrumpf kann nun über den Hohlkörper gleitend auf das Bein aufgeschoben werden. Nachdem der Stützstrumpf seine Sollposition erreicht hat, d.h. den vorgesehenen Bereich des Beines faltenfrei umschließt, wird die An-und Ausziehhilfe wieder entfernt, dazu genügt es, durch die freie Fußspitze des Stützstrumpfs in dem Bereich in dem die Tasche ihr geschlossenes Ende hat an der Anziehhilfe zu ziehen. Die Anziehhilfe kann dann durch die offene Fußspitze aus dem Stützstrumpf entfernt werden einfach herausgezogen werden, wozu wie nachstehend noch näher erläutert vorzugsweise ein Griffstück, z.B. in Form einer Öse, im Bereich des geschlossenen Endes der Tasche angebracht sein kann.

Zum Ausziehen eines Stützstrumpfs wird der Fuß (mit Strumpf) wieder in die Tasche eingeschoben, wobei das freie Ende des zweiten Streifens vorzugsweise auf dem Fuß, bzw. an der Vorderseite des Beines zu liegen kommt. Nun wird der Fuß mit der Tasche und damit das Bein in den Hohlkörper eingeschoben, bis der Hohlkörper zumindest im Wesentlichen den Stützstrumpf zumindest im Bereich des Beines umgibt (d.h. der Fußbereich ist nicht notwendigerweise im Hohlkörper). Der Hohlkörper sitzt nun schlauchartig auf dem Stützstrumpf. Anschließend wird der obere Rand des Stützstrumpfs nach außen umgeschlagen, so dass er außen auf dem Hohlkörper zu liegen kommt. Der Stützstrumpf bildet nun eine umlaufende Tasche, die den Hohlköper aufnimmt. Dieser umgeschlagene Rand, kann nun über die gleitende Oberfläche des Hohlkörpers in Richtung der Zehen verschoben werden. Dabei 'rollt' das geschlossene Ende der umlaufenden Tasche über den Hohlkörper ab und nimmt diesen dabei mit. Folglich liegt der Hohlkörper auf sich selbst auf und der Stützstrumpf kann sehr einfach nach unten abgezogen werden.

Vorzugsweise ist zwischen dem geschlossene Ende der Tasche und dem Hohlkörper wenigstens ein weiterer Unterabschnitt angeordnet, in dem die Breite des zweiten Abschnitts in Richtung des Hohlkörpers zunimmt. Dadurch wird mit sehr geringen Kosten ein Übergangsbereich geschaffen, in dem Zugkräfte gleichmäßig von dem zweiten Abschnitt in den Bereich des Hohlkörpers eingebracht werden können und vice versa.

Wenn zwischen dem ersten Unterabschnitt und dem weiteren Unterabschnitt wenigstens ein dazwischenliegender Unterabschnitt angeordnet ist, der eine zumindest in etwa konstante Breite aufweist, ist die An- und Ausziehhilfe besonders einfach fertigbar, da die Länge durch Variation der Länge des dazwischenliegenden Unterabschnitts leicht angepasst werden kann, ohne das Schnittmuster für die An- und Ausziehhilfe in sonstiger Weise anpassen zu müssen.

Beispielsweise kann der erste Unterabschnitt in der Aufsicht zwei sich in quer zur Längsachse der Streifen flügelartig erweiternde Fortsätze, d.h. Ansätze haben. Die Randbereiche der Ansätze können miteinander verbunden sind, wodurch der Hohlkörper gebildet wird. Dadurch lässt sich der Hohlkörper sehr einfach und somit kostengünstig fertigen und die Anzahl der zuzuschneiden Teile ist minimal (nämlich nur zwei, wenn die beiden Streifen jeweils einteilig sind). Wenn der erste Unterabschnitt an nur einem seitlichen Rand einen flügelartigen Fortsatz hat, dann kann in gleicher Weise der Hohlkörper gebildet werden, jedoch ist die Verbindungsnaht dann nicht mittig sondern entsprechend seitlich versetzt.

Vorzugsweise verjüngt sich die von dem Hohlkörper eingeschlossene freie Querschnittsfläche in Richtung des freien Endes des zweiten Abschnitts. Dadurch passt sich die An- und Ausziehhilfe besonders gut der Form des Beines an und vor allem kann die An- und Ausziehhilfe nicht am Bein hochrutschen. Sie muss also nicht mit den Zehen festgehalten werden, was gerade älteren Menschen oft nicht zu leisten vermögen.

Wenn sich die freie Querschnittsfläche monoton verjüngt, wird die Fertigung der An- und Ausziehhilfe vereinfacht.

Vorzugsweise ist an dem von der Tasche wegweisenden Ende des Hohlkörpers das zuvor schon genannte Griffstück angeordnet, wodurch das Trennen von Stützstrumpf und An- und Ausziehhilfe nach dem Ausziehen vereinfacht wird, insbesondere bei der Verwendung vorne offener Stützstrümpfe.

Das Griffstück kann einstückig mit dem ersten Abschnitt ausgebildet sein, so dass neben dem zusätzlichen Materialverbrauch keine weiteren Fertigungskosten durch das Griffstück entstehen.

Zur Verstärkung kann ein sich in Längsrichtung erstreckender Gewebestreifen den Übergang zwischen dem Griffstück und dem ersten Unterabschnitt überbrücken.

Wie schon aus dem Vorstehenden ersichtlich, wird der Hohlkörper durch den ersten folienartigen Streifen ausgebildet, der an seinen Seitenrändern an einem Unterabschnitt des zweiten Abschnitts zusammengefügt ist. Der Hohlkörper ist folglich nicht starr sondern ähnelt diesbezüglich einem Windsack, der wie ein Durchgangstunnel zwei offene Enden hat.

### Beschreibung der Zeichnungen

Die Erfindung wird nachstehend ohne Beschränkung des allgemeinen Erfindungsgedankens anhand von Ausführungsbeispielen unter Bezugnahme auf die Zeichnungen exemplarisch beschrieben.
Figur 1 zeigt eine An- und Ausziehhilfe.
Figur 2 zeigt die An- und Ausziehhilfe vor der Fertigstellung.
Figur 3 zeigt die An- und Ausziehhilfe in Verwendung.

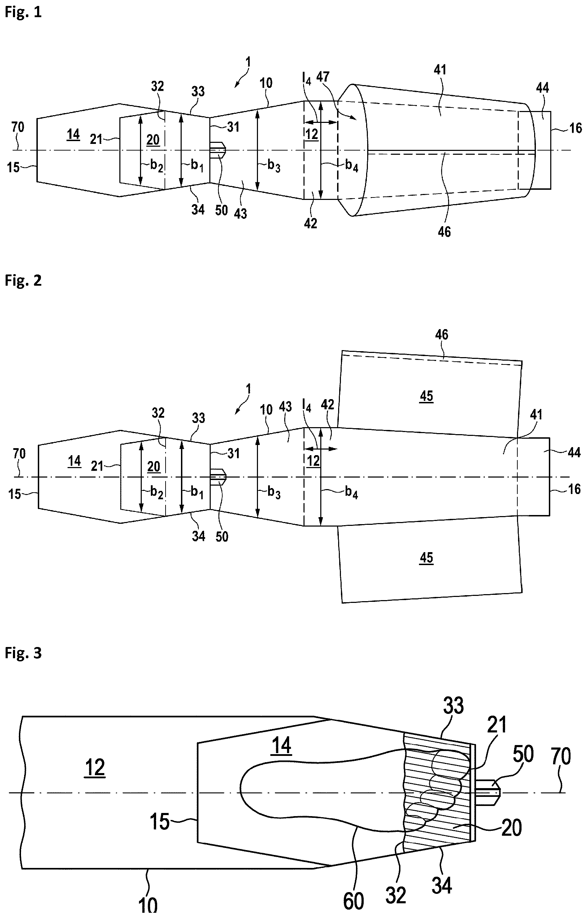

Die Anziehhilfe 1 für Kompressionsstrümpfe in Figur 1 hat einen ersten Streifen 10 mit einer Längsachse 70, die eine Längsrichtung (genauer zwei zueinander diametrale Längsrichtungen) definiert. Der erste Streifen 10 ist durch eine weiter unten erläuterte Verbindung 31 in einen ersten Abschnitt 12 und einen relativ dazu bevorzugt kürzeren zweiten Abschnitt 14 unterteilt. Im gezeigten Beispiel ist der erste Streifen 10 einstückig, aber natürlich kann er auch aus aneinander befestigten Abschnitten wie 12, 14 gefertigt sein. Auf den zweiten Abschnitt 14 ist ein zweiter Streifen 20 aufgesetzt, so dass er mit einem Segment des zweiten Abschnitts 14 eine Tasche bildet. Die Streifen 10, 20 sind folglich miteinander verbunden (z.B. miteinander vernäht und/oder verklebt und/oder verschweißt) wobei die Verbindung den Rand der Tasche definiert. Die Tasche wird in Längsrichtung zum einen durch die Verbindung 31 und eine der Verbindung 31 gegenüberliegende Öffnung 32 begrenzt. Die Verbindung 31 ist somit das in Längsrichtung geschlossene Ende der Tasche und unterteilt gleichzeitig den ersten Streifen 10 in die beiden Abschnitte. Die Verbindung 31 kann beispielsweise genäht und/oder geschweißt und/oder geklebt sein. Auch seitlich ist die Tasche geschlossen, d.h. an den Kanten 33 und 34 sind der erste Streifen 10 und der zweite Streifen 20 miteinander verbunden. Die freien Enden 21, 15 des zweiten Streifens 10 bzw. des zweiten Abschnitts 14 stehen über die Öffnung 32 der Tasche über. Der Abstand b₁ der beiden Kanten 33 und 34 zueinander nimmt in Richtung der Öffnung 32 zu; dadurch lässt sich die Anziehhilfe besonders leicht durch die Einstiegsöffnung aus dem Kompressionsstrumpf herausziehen. Die Breite b₂ des freien zungenartigen Endes 21 des zweiten Streifens 20 nimmt mit zunehmendem Abstand von der Öffnung 32 ab, wodurch es sich besonders gut in die Tasche einstecken lässt. Ein optionales als Öse ausgebildetes Griffstück 50 kann beispielsweise im Bereich der Verbindung 31 an zumindest einem der beiden Streifen 10, 20 befestigt sein. Auch die Breite des freien Endes 15 des zweiten Abschnitts 14 des ersten Streifens 10 nimmt mit zunehmendem Abstand von der Öffnung 32 ab. Dadurch lässt sich auch das freie Ende 15 des zweiten Abschnitts 14 in die Tasche einschlagen, was besonders vorteilhaft ist, wenn die Anziehhilfe 1 durch eine offene Fußspitze aus einem Kompressionsstrumpf gezogen wird.

Der erste Abschnitt 12 des ersten Streifens 10 hat Unterabschnitte 41, 42, 43 und 44. Ein erster der Unterabschnitte 41 ist als Hohlkörper 41 ausgebildet. In diesem Bereich hat der erste Streifen 10 flügelartige Ansätze 45 (vgl. Fig. 2) deren Längsseiten miteinander verbunden sind, so dass der erste Unterabschnitt 41 einen sich entlang der Längsachse 70 erstreckenden beidseits offenen Tunnel 47 umschließt.

In der Figur 2 ist an einem der beiden flügelartigen Ansätzen 45 eine ,Nahtzugabe 46' mit einer gestrichelten Line angedeutet. Entsprechend überlappen sich die beiden flügelartigen Ansätze 45 in ihren Randbereichen (durch gestrichelte Linie in Fig. 1 angedeutet). Alternativ könnten auch die Längskanten (streng genommen die Längsseiten, die hier wegen ihrer geringen Dicke als Kante bezeichnet werden) stumpf zusammengefügt werden, z.B. durch Stoßschweißen. Der mit 44 gekennzeichnete (optionale) Unterabschnitt 44 sitzt ausgangsseitig des Tunnels 47 und kann als Griffstück dienen.

Ein weiterer (optionaler) Unterabschnitt 43 ist zwischen dem ersten Unterabschnitt 41 und dem geschlossenen Ende 31 der Tasche angeordnet. In diesem Unterabschnitt verbreitert sich der erste Streifen 10 von dem Ende 31 der Tasche monoton in Richtung des freien Endes 16 des ersten Abschnitts 12 so dass Zugkräfte in Längsrichtung zwischen dem Rand 31 der Tasche und dem ersten Unterabschnitt 41 gleichmäßig eingeleitet werden. In anderen Worten, die Breite b₃ des weiteren Unterabschnitts 43 nimmt in Richtung des freien Endes 16 zu. Zwischen dem weiteren Unterabschnitt 43 und dem ersten Unterabschnitt 41 kann ein dritter Unterabschnitt 42 angeordnet sein, in dem die Breite b₄ des ersten Streifens 10 zumindest in etwa konstant bleibt. Die Länge l₄ es dritten Unterabschnitts 42 kann zur Einstellung der Gesamtlänge des ersten Abschnitts 12 sehr einfach variiert werden.

Figur 3 zeigt die Anziehhilfe kurz bevor ein Kompressionsstrumpf (nicht dargestellt) über ein durch einen Fußabdruck 60 angedeutetes Bein gezogen werden kann. Dazu wurde der erste Abschnitt 12 entlang der Verbindung 31 umgeschlagen, so dass er unter dem zweiten Abschnitt 14 angeordnet ist. Das Griffstück 50 steht nun frei. Zudem wurde das freie Ende 21 des zweiten Streifens 20 in die Tasche umgeschlagen, was durch eine Schraffur angedeutet ist. Danach wurde der Vorfuß des Beins 60 wie angedeutet durch die Öffnung 32 in die Tasche eingeführt. Als nächstes wäre ein nicht dargestellter Kompressionsstrumpf über die Tasche und das Bein zu ziehen, wobei sich der Kompressionsstrumpf vergleichsweise leicht über das folienartige Material und somit über den Fuß und einen sich daran anschließenden Unterschenkel (nicht dargestellt) anziehen lässt. Anschließend wäre die Anziehhilfe 1 aus dem Kompressionsstrumpf zu ziehen. Dazu könnte man sie am freien Ende des ersten Abschnitts 12 des ersten Streifens 10 aus dem Kompressionsstrumpf herausziehen, wobei das freie Ende 21 des zweiten Streifens 20 aus der Tasche herausgeklappt würde, was es erleichtern würde den zweiten Streifen 20 über die Zehen zu ziehen. Alternativ könnte man die Anziehhilfe - sofern der Kompressionsstrumpf im Bereich der Zehen ein Kontrollloch hätte - am aus dem Kontrollloch herausgeführten Griffstück 50 durch das Kontrollloch aus dem Kompressionsstrumpf ziehen.

### Bezugszeichenliste

- 1: Anziehhilfe
- 10: erster Streifen
- 12: erster Abschnitt des ersten Streifens
- 14: zweiter Abschnitt des ersten Streifens
- 15: freies Ende des zweiten Abschnitts 14
- 16: freies Ende des ersten Abschnitts 12
- 20: zweiter Streifen
- 21: freies Ende des zweiten Streifens
- 31: geschlossenes Ende
- 32: Öffnung
- 33: Kante
- 34: Kante
- 41: erster Unterabschnitt des ersten Abschnitts 12 / Hohlkörper
- 42: Unterabschnitt des ersten Abschnitts 12
- 43: Unterabschnitt des ersten Abschnitts 12
- 44: Unterabschnitt des ersten Abschnitts 12
- 45: seitlicher Ansatz, Fortsatz
- 47: Tunnel (beidseits offen)
- 50: Griffstück
- 60: Bein
- 70: Längsachse
- b₁: Breite der Tasche
- b₂: Breite des die Öffnung 32 überragenden Abschnitts des zweiten Streifens 20
- b₃: Breite des Unterabschnitts 43
- b₄: Breite des Unterabschnitts 42
- l₄: Länge des Unterabschnitts 42

## Patentansprüche

1. An- und Ausziehhilfe (1) für Kompressionsstrümpfe mit wenigstens einem ersten folienartigen Streifen (10), wobei auf den ersten Streifen (10) ein zweiter Streifen (20) derart aufgesetzt ist, dass er mit dem ersten Streifen (10) eine Tasche mit einem in Längsrichtung weisenden geschlossenen Ende (31) bildet, die an dem dem geschlossenen Ende (31) gegenüberliegenden Ende eine Öffnung (32) in Längsrichtung hat, wobei
- die Öffnung (32) vom ersten Streifen (10) und vom zweiten Streifen (20) in Längsrichtung überragt wird, und
- das geschlossene Ende (31) der Tasche den ersten Streifen (10) in zwei Abschnitte (12, 14) unterteilt, wobei die Tasche auf dem zweiten Abschnitt (14) angeordnet ist,
**dadurch gekennzeichnet, dass**
der erste Abschnitt (12) wenigsten einen ersten Unterabschnitt (41) hat, an dem seine beiden Längsseiten miteinander verbunden sind, so dass der Unterabschnitt (41) einen Hohlkörper (41) bildet.

2. An- und Ausziehhilfe (1) für Kompressionsstrümpfe nach Anspruch 1,
**dadurch gekennzeichnet, dass**
zwischen dem geschlossene Ende der Tasche und dem Hohlkörper (41) wenigstens ein weiterer Unterabschnitt (43) angeordnet ist, in dem die Breite (b₃) des ersten Abschnitts (12) in Richtung des Hohlkörpers zunimmt.

3. An- und Ausziehhilfe für Kompressionsstrümpfe nach Anspruch 1 oder 2,
**dadurch gekennzeichnet, dass**
zwischen dem ersten Unterabschnitt (41) und dem weiteren Unterabschnitt (43) wenigstens ein dazwischenliegender Unterabschnitt (42) angeordnet ist, der eine zumindest in etwa konstante Breite (b₄) aufweist.

4. An- und Ausziehhilfe für Kompressionsstrümpfe nach einem der vorstehenden Ansprüche,
**dadurch gekennzeichnet, dass**
der erste Unterabschnitt (41) in der Aufsicht zwei sich quer zur Längsachse (70) flügelartig erweiternde Fortsätze (45) hat, deren Randbereiche miteinander verbunden sind, wodurch der Hohlkörper (41) gebildet wird.

5. An- und Ausziehhilfe für Kompressionsstrümpfe nach einem der vorstehenden Ansprüche, **dadurch gekennzeichnet, dass**
die von dem Hohlkörper eingeschlossene freie Querschnittsfläche sich in Richtung des freien Endes des zweiten Abschnitts (12) verjüngt.

6. An- und Ausziehhilfe für Kompressionsstrümpfe nach Anspruch 5,
**dadurch gekennzeichnet, dass**
sich die freie Querschnittsfläche monoton verjüngt.

7. An- und Ausziehhilfe für Kompressionsstrümpfe nach einem der vorstehenden Ansprüche, **dadurch gekennzeichnet, dass**
das an dem von der Tasche wegweisenden Ende des Hohlkörpers ein Griffstück angeordnet ist.

8. An- und Ausziehhilfe für Kompressionsstrümpfe nach Anspruch 7,
**dadurch gekennzeichnet, dass**
das Griffstück einstückig mit dem ersten Abschnitt (12) ausgebildet ist.

9. An- und Ausziehhilfe für Kompressionsstrümpfe nach Anspruch 7 oder 8,
**dadurch gekennzeichnet, dass**
ein sich in Längsrichtung erstreckender Gewebestreifen den Übergang zwischen dem Griffstück und dem ersten Unterabschnitt (41) überbrückt.

## Claims

1. Dressing and undressing aid (1) for compression stockings with at least one first film-like strip (10), wherein a second strip (20) is placed on the first strip (10) such that it forms a pocket with the first strip (19), with a closed end (31) oriented in longitudinal direction, said pocket having an opening (32) in longitudinal direction at the end opposite to the closed end (31), wherein
- the first strip (10) and the second strip (20) extend in the longitudinal direction over the opening (32), and
- the closed end (31) of the pocket divides the first strip (10) into two sections (12, 14), wherein the pocket is arranged on the second section (14),
**characterized in that**
the first section (12) has at least a first subsection (41) at which its two longitudinal sides are connected to one another, such that the subsection (41) forms a hollow body (41).

2. Dressing and undressing aid (1) for compression stockings according to claim 1,
**characterized in that**
between the closed end of the pocket and the hollow body (41) at least one further subsection (43) is arranged, in which the width (b₃) of the first section (12) increases in the direction of the hollow body.

3. Dressing and undressing aid for compression stockings according to claim 1 or 2,
**characterized in that**
between the first subsection (41) and the further subsection (43) at least one intermediate subsection (42) is arranged, which has an at least approximately constant width (b₄).

4. Dressing and undressing aid for compression stockings according to one of the preceding claims,
**characterized in that**
the first subsection (41) has two extensions (45) in the plan view, that extend in a wing-like manner transversely to the longitudinal axis (70), the edge regions of said extensions being connected to one another, whereby the hollow body (41) is formed.

5. Dressing and undressing aid for compression stockings according to one of the preceding claims,
**characterized in that**
free cross-sectional area enclosed by the hollow body tapers in the direction of the free end of the second section (12).

6. Dressing and undressing aid for compression stockings according to claim 5,
**characterized in that**
the free cross-sectional area tapers monotonically.

7. Dressing and undressing aid for compression stockings according to one of the preceding claims,
**characterized in that**
a handle is arranged at the end of the hollow body, which end faces away from the pocket.

8. Dressing and undressing aid for compression stockings according to claim 7,
**characterized in that**
the handle is formed integrally with the first section (12).

9. Dressing and undressing aid for compression stockings according to claim 7 or 8,
**characterized in that**
a fabric strip extending longitudinally bridges the transition between the handle and the first subsection (41).

## Revendications

1. Aide (1) pour enfiler et ôter des bas de contention avec au moins une première bande (10) en forme de feuille, avec une deuxième bande (20) posée sur la première bande (10) de façon à former avec la première bande (10) une poche avec une extrémité fermée (31) orientée dans le sens de la longueur, qui présente une ouverture (32) dans le sens de la longueur à l'extrémité opposée à l'extrémité fermée (31), dans laquelle
- la première bande (10) et la deuxième bande (20) dépassent de l'ouverture (32) dans le sens de la longueur et
- l'extrémité fermée (31) de la poche divise la première bande (10) en deux sections (12, 14), la poche étant disposée sur la deuxième section (14),
**caractérisée en ce que** la première section (12) présente au moins une première sous-section (41) dont les deux côtés longitudinaux sont reliés ensemble de sorte que la sous-section (41) forme un corps creux (41).

2. Aide (1) pour enfiler et ôter des bas de contention selon la revendication 1, **caractérisée en ce qu'**est disposée entre l'extrémité fermée de la poche et le corps creux (41) au moins une autre sous-section (43) dans laquelle la largeur (b₃) de la première section (12) diminue en direction du corps creux.

3. Aide pour enfiler et ôter des bas de contention selon la revendication 1 ou 2, **caractérisée en ce qu'**est disposée entre la première sous-section (41) et l'autre sous-section (43) au moins une sous-section (42) intercalée qui présente une largeur (b₄) au moins à peu près constante.

4. Aide pour enfiler et ôter des bas de contention selon l'une des revendications précédentes, **caractérisée en ce que** la première sous-section (41) présente, en vue de dessus, deux saillies (45) qui s'élargissent en forme d'ailes transversalement par rapport à l'axe longitudinal (70) et dont les zones de bord sont reliées entre elles de façon à former le corps creux (41).

5. Aide pour enfiler et ôter des bas de contention selon l'une des revendications précédentes, **caractérisée en ce que** l'aire de section libre renfermée par le corps creux se resserre en direction de l'extrémité libre de la deuxième section (12).

6. Aide pour enfiler et ôter des bas de contention selon la revendication 5, **caractérisée en ce que** l'aire de section libre se resserre de façon régulière.

7. Aide pour enfiler et ôter des bas de contention selon l'une des revendications précédentes, **caractérisée en ce qu'**une poignée est disposée à l'extrémité du corps creux opposée à la poche.

8. Aide pour enfiler et ôter des bas de contention selon la revendication 7, **caractérisée en ce que** la poignée est formée d'un seul tenant avec la première section (12).

9. Aide pour enfiler et ôter des bas de contention selon la revendication 7 ou 8, **caractérisée en ce qu'**une bande de textile qui s'étend dans le sens de la longueur couvre la transition entre la poignée et la première sous-section (41).
